# EUROPÄISCHE PATENTSCHRIFT

(11) **EP 0 656 058 B1**
(45) Veröffentlichungstag und Bekanntmachung des Hinweises auf die Patenterteilung: **10.12.1997**
(21) Anmeldenummer: 93917747.3
(22) Anmeldetag: 05.08.1993
(51) Int. Cl.: C12N 9/96, C12N 9/98, C11D 3/386

(54) **NEUE ENZYMGRANULATE**
NOVEL ENZYME GRANULATES
NOUVEAUX GRANULES D'ENZYMES

(30) Priorität: 14.08.1992 DE 4226923
(43) Veröffentlichungstag der Anmeldung: 07.06.1995
(73) Patentinhaber: Genencor International GmbH, 31582 Nienburg/Weser (DE)
(72) Erfinder: KIESSER, Torsten, W., D-31852 Nienburg/Weser (DE); HERRMAN, Hubert, A., D-31852 Nienburg/Weser (DE); KONIECZNY-JANDA, Gerhard, D-30982 Pattensen (DE)
(74) Vertreter: Wibbelmann, Jobst, Dr., Dipl.-Chem.
(86) Internationale Anmeldenummer: EP9302081
(87) Internationale Veröffentlichungsnummer: WO9404665

(56) Entgegenhaltungen:
- EP-A- 0 170 360
- EP-A- 0 256 127
- WO-A-91/09941
- WO-A-91/09943
- WO-A-92/11347
- DE-A- 2 831 778
- GB-A- 2 186 883

## Beschreibung

Die vorliegende Erfindung betrifft neue Enzymgranulate, ein Verfahren zu deren Herstellung sowie deren Verwendung in Wasch- und Reinigungsmittelzusammensetzungen.

In zahlreichen Waschmittelzusammensetzungen, z. B. zur Reinigung von Textilien oder Geschirr, werden Enzyme zur Erhöhung der Waschwirksamkeit eingesetzt. Üblicherweise verwendet man dabei als Enzyme Proteasen, Lipasen, Amylasen oder Cellulasen. Pulverförmigen Waschmittelformulierungen werden die Enzyme in Form sogenannter Enzymgranulate, die das betreffende Enzym oder Enzymgemisch zusammen mit einem Füllstoff und gegebenenfalls weiteren Granulierhilfsstoffen enthalten, beigefügt. Derartige Enzymgranulate werden üblicherweise dadurch erhalten, daß man ein Enzymkonzentrat mit Füll- und Bindestoffen sowie gegebenenfalls noch weiteren Granulierhilfsstoffen zu einer Masse verarbeitet und granuliert. Die erhaltenen Granulatpartikel werden anschließend getrocknet, gegebenenfalls mit einem Schutzlack überzogen und können dann in pulverförmigen Waschmittelformulierungen eingesetzt werden.

Es besteht weiterhin Bedarf nach Enzymgranulaten, die eine insbesondere auch in Gegenwart üblicher Waschmittelbestandteile verbesserte Lagerstabilität und gute Lösungseigenschaften besitzen.

Es bestand daher die Aufgabe, neue Enzymgranulate zur Verfügung zu stellen, die eine hohe Lagerstabilität und gute Lösungseigenschaften aufweisen und die sich insbesondere zur Verwendung in pulverförmigen Waschmittelformulierungen eignen.

Es wurden nun Enzymgranulate gefunden, die die geforderten Eigenschaften zeigen.

Gegenstand der Erfindung ist daher ein Enzymgranulat, enthaltend ein Enzym oder Enzymgemisch, ein wasserunlösliches Füllstoffgemisch, wasserlösliche Füllstoffgemische, Bindemittel sowie gegebenenfalls weitere Granulierhilfsstoffe, wobei es ein Alkali- oder Erdalkaliformiat und gegebenenfalls reduzierende Zucker enthält.

Durch den Zusatz eines Alkali- oder Erdalkaliformiats, wird die Lagerstabilität der in den erfindungsgemäßen Granulaten verarbeiteten Enzyme in überraschender Weise erhöht, wobei die stabilisierende Wirkung des Alkali- oder Erdalkaliformiats gewünschtenfalls durch den Zusatz reduzierender Zucker in synergistischer Weise noch weiter verstärkt werden kann. Bevorzugt setzt man daher das Alkali- oder Erdalkaliformiat in den erfindungsgemäßen Granulaten immer in Kombination mit reduzierenden Zuckern ein.

Vorzugsweise sollte die Menge an Alkali- oder Erdalkaliformiat in den erfindungsgemäßen Enzymgranulaten 0,5 bis 15 Gew.-%, vorzugsweise 1 bis 8 Gew.-%, bezogen auf den Gesamtfeststoffgehalt, betragen. Erdalkaliformiate können dabei entweder als solche zugesetzt werden oder in situ aus einem Alkalimetallformiat, insbesondere Natriumformiat, und einem wasserlöslichem Erdalkalimetallsalz, insbesondere einem Calciumsalz, gebildet werden. Besonders bevorzugt setzt man in den erfindungsgemäßen Enzymgranulaten Calciumformiat in den angegebenen Mengen ein.

Sollen gegebenenfalls zur synergistischen Verstärkung der stabilisierenden Wirkung des in den erfindungsgemäßen Enzymgranulaten eingesetzten Alkali- oder Erdalkaliformiats reduzierende Zucker verwendet werden, so setzt man vorteilhaft als reduzierende Zucker Monosaccharide wie Glukose oder Dissaccharide wie Lactose und/oder Maltose oder Polysaccharide wie Dextrine ein. Geeignet sind auch Gemische aus reduzierenden Zuckern wie z. B. Glukosesirup. Bevorzugt werden Disaccharide, insbesondere Lactose und/oder Maltose in den erfindungsgemäßen Enzymgranulaten verwendet. Vorteilhaft werden die vorstehend genannten reduzierenden Zucker in den erfindungsgemäßen Enzymgranulaten in einer Menge von 0,5 bis 20 Gew.-%, insbesondere 2,0 bis 10,0 Gew.-%, bezogen auf den Gesamtfeststoffgehalt, eingesetzt.

Als Enzyme können in den erfindungsgemäßen Enzymgranulaten alle in Wasch- und Reinigungsmittelzusammensetzungen üblichen Enzyme, beispielsweise Enzyme wie Proteasen, Lipasen, Amylasen, Glucanasen wie Cellulasen, Hemicellulasen, Pullulanasen, oder Oxidoreduktasen, vorzugsweise Amylasen, Cellulasen, Lipasen oder Proteasen enthalten sein. Die Enzyme können in den erfindungsgemäßen Enzymgranulaten einzeln oder auch als Enzymgemische, beispielsweise als Protease/Amylase-Gemische oder Protease/Lipase-Gemische enthalten sein.

In einer besonders bevorzugten Ausgestaltung enthalten die erfindungsgemäßen Enzymgranulate Proteasen, insbesondere alkalische Proteasen. In vorteilhafter Weise können dabei besonders auch die Proteasen in die erfindungsgemäßen Enzymgranulate eingearbeitet werden, die verbesserten Eigenschaften wie gesteigerte Waschleistung oder verbesserte Stabilität aufgrund chemischer und/oder gentechnischer Modifikationen besitzen. Hierbei sind als alkalische Proteasen insbesondere die sogenannten Subtilisine vorteilhaft. Subtilisine sind alkalische Proteasen mit einem pH-Optimum im alkalischen pH-Bereich und einen essentiellen Serinrest im aktiven Zentrum. Sie können auf an sich bekannte Weise aus Gram-positiven Bakterien oder Pilzen gewonnen werden. Bevorzugt sind dabei die aus Bacillus-Stämmen gewonnene Subtilisine, beispielsweise Subtilisine wie Subtilisin BPN'-, Subtilisin Carlsberg und die Subtilisine, die aus Bacillus Subtilis, Bacillus amyloliquefaciens, Bacillus licheniformis, Bacillus lentus, Bacillus mesentericus oder Bacillus alcalophilus isoliert werden können. Besonders bevorzugt sind Subtilisine, die ein pH-Optimum im Bereich von 7-13 haben und die z. B. als Savinase^{R}, Maxacal^{R}, Durazym^{R}, Maxapem^{R} oder Opticlean^{R} kommerziell erhältlich sind.

Die für die erfindungsgemäßen Enzymgranulate geeigneten Enzyme können auf an sich bekannte Weise durch Fermentationsprozesse aus geeigneten Mikroorganismen, insbesondere aus Bakterien oder Pilzen, gewonnen werden. Die bei der Fermentation erhaltenen Fermenterbrühen werden von unlöslichen Begleitstoffen, z. B. durch Filtration, befreit und anschließend auf an sich bekannte Weise, z. B. durch Membranfiltrationsverfahren wie Mikro- und/ oder Ultrafiltration mit gegebenenfalls sich anschließender Dialyse und/oder durch Dünnschichtverdampfung eingeengt. Man erhält somit sogenannte Enzymkonzentrate, die üblicherweise das Enzym bzw. Enzymgemisch in einer Menge von 2 bis 50 Gew.-%, bezogen auf die Trockensubstanz, neben eventuell weiter nicht abgetrennten Begleitstoffen enthalten. Gewünschtenfalls können diese flüssigen Enzymkonzentrate noch weiter, beispielsweise durch Sprühtrocknung, und/oder Gefriertrocknung in trockene Enzymkonzentrate überführt werden.

Das im erfindungsgemäßen Enzymgranulat enthaltene wasserunlösliche Füllstoffgemisch besteht üblicherweise aus einem Gemisch aus Cellulose und einem Schichtsilikat oder Schichtsilikatgemisch, wobei in einer bevorzugten Ausführung gegebenenfalls noch Getreidemehl und/oder Stärke weitere Bestandteile des wasserunlöslichen Füllstoffgemisches sind. Getreidemehl und Stärke können dabei entweder allein oder im Gemisch miteinander eingesetzt werden. Als Getreidemehl können dabei alle an sich bekannte Mehle aus Weizen, Roggen, Gerste, Hafer, Reis oder Mais verwendet werden. Vorzugsweise wird Weizenmehl eingesetzt.

Der Cellulose-Bestandteil im wasserunlöslichen Füllstoffgemisch besteht aus faserförmiger Cellulose, beispielsweise aus faserförmiger Cellulose mit einer mittleren Faserlänge im Bereich von etwa 20 bis 50 µm. Als besonders bevorzugt geeignet hat sich faserförmige Cellulose mit einer mittleren Faserlänge von etwa 30 µm erwiesen.

Als weitere Komponente enthält das wasserunlösliche Füllstoffgemisch Schichtsilikate wie Bentonit oder Kaolin oder Schichtsilikatgemische aus Kaolin und Bentonit. Kaolin kann gegebenenfalls auch im Gemisch mit Calciumcarbonat und/oder Bentonit enthalten sein.

Als wasserlösliche Füllstoffgemische sind üblicherweise anorganische wasserlösliche Salze wie z.B. Alkalichloride, Alkaliacetate, Alkalisulfate oder deren Gemische in den erfindungsgemäßen Enzymgranulaten enthalten. Bevorzugt sind die Alkalisulfate, insbesondere Natriumsulfat.

Als Bindemittel enthalten die erfindungsgemäßen Enzymgranulate Bindemittel aus der Gruppe Polyethylenglykol, insbesondere Polyethylenglykole mit Molekulargewichten im Bereich von 200 bis 10.000, und/oder Polyvinylpyrrolidon, insbesondere Polyvinylpyrrolidon mit Molekulargewichten im Bereich von 12.000 bis 3.000.000, vorzugsweise 1.300.000 bis 2.800.000. Dabei kann gegebenenfalls als Bindemittel nur Polyethylenglykol zugesetzt sein.

Die erfindungsgemäßen Enzymgranulate können, jeweils bezogen auf den Gesamtfeststoffgehalt, sich beispielsweise so zusammensetzen, daß, das wasserunlösliche Füllstoffgemisch aus 15 bis 40 Gew.-% Cellulose, 5 bis 18 Gew.-% Kaolin, gewünschtenfalls im Gemisch mit 1 bis 35 Gew.-% Bentonit und 1 bis 13 Gew.-% Calciumcarbonat besteht. Gegebenenfalls können als wasserunlösliche Füllstoffe noch 0 bis 10 Gew.-% Getreidemehl und/oder 0 bis 50 Gew.-% Stärke enthalten sein. Als wasserlösliche Füllstoffe werden üblicherweise 0,5 bis 20 Gew.-% Natriumsulfat eingesetzt. Weiterhin können bis zu 30 Gew.-% Bindemittel aus der Gruppe Polyethylenglykol und Polyvinylpyrrolidon enthalten sein. Das Enzym kann als Enzymkonzentrat in einer Menge von 1 bis 40 Gew.-%, das auf an sich bekannte Weise, wie vorstehend beschrieben, hergestellt wurde, in den erfindungsgemäßen Enzymgranulaten vorliegen. Zur Stabilisierung der Enzyme können erfindungsgemäß die Granulate 0,5 bis 15 Gew.-% Alkali- oder Erdalkaliformiat und gegebenenfalls 0,5 bis 20 Gew.-% reduzierende Zucker, insbesondere reduzierende Disaccharide, enthalten.

In einer bevorzugten Ausgestaltung enthält ein erfindungsgemäßes Enzymgranulat, jeweils bezogen auf den Gesamtfeststoffgehalt, 10 bis 20 Gew.-% Enzymkonzentrat, 20 bis 30 Gew.-% Cellulose, 0 bis 8 Gew.-% Getreidemehl und/oder 0 bis 20 Gew.-% Stärke, 10 bis 15 Gew.-% Kaolin, 5 bis 12 Gew.-% Calciumcarbonat, 10 bis 15 Gew.-% Natriumsulfat, 0,5 bis 2 Gew.-% Polyvinylpyrrolidon (Molekulargewicht 1.300.000), 10 bis 15 Gew.-% Polyethylenglykol (Molekulargewicht 3000), 2,0 bis 10 Gew.-% Lactose sowie 1 bis 8 Gew.-% Calciumformiat.

Selbstverständlich können in den erfindungsgemäßen Enzymgranulaten neben den vorgenannten Hauptbestandteilen noch weitere Granulierhilfsstoffe wie z. B. Gleitmittel oder Dispergierhilfsstoffe enthalten sein. Als Gleitmittel kann z.B. ein Glycerinmonoester mit langkettigen Fettsäuren, als Dispergierhilfsstoff beispielsweise ein Sulfobernsteinsäureester mit langkettigen Fettalkoholen in Konzentrationen von bis zu 10 Gew.-% verwendet werden.

Weiterhin umfaßt die Erfindung ein Verfahren zur Herstellung von Enzymgranulaten, bei dem man eine durch Vermischen eines Enzymkonzentrates mit Wasser, Füllstoffen, Bindemitteln sowie gegebenenfalls weiteren Bestandteilen erhaltene extrudierbare Masse zu Partikel extrudiert, die erhaltenen Partikel in einem Rundungsgerät rundet, die gerundeten Partikel anschließend trocknet sowie gegebenenfalls mit einem Schutzlack überzieht, wobei man der extrudierbaren Masse, bezogen auf den Gesamtfeststoffgehalt des Enzymgranulates, ein Alkali- oder Erdalkaliformiat, vorzugsweise Calciumformiat in einer Menge von 0,5 bis 15 Gew.-% und gegebenenfalls reduzierende Zucker, vorzugsweise Lactose und/oder Maltose, in einer Menge von 0,5 bis 20 Gew.-% zugibt.

In dem erfindungsgemäßen Verfahren können als Enzymkonzentrate flüssige Enzymkonzentrate, wie sie auf an sich bekannte Weise durch Fermentation von Mikroorganismen und Aufarbeiten der bei der Fermentation anfallenden Fermenterbrühen erhalten werden, eingesetzt werden. Unter Enzymkonzentraten im Sinne der Erfindung werden aber auch feste Enzymkonzentrate verstanden, wie sie beispielsweise durch Gefriertrocknung von flüssigen Enzymkonzentraten gewonnen werden können.

Zweckmäßigerweise wird das Verfahren so durchgeführt, daß das Enzymkonzentrat einer zuvor hergestellten trockenen Vormischung der übrigen pulverförmigen Rezeptbestandteile in einem geeigneten Mischgerät, beispielsweise einem Konusmischer oder Pflugscharmischer, zugesetzt wird. Dann wird soviel Wasser zudosiert, daß sich eine gut verformbare und extrudierbare Masse bildet. Üblicherweise liegt der Feuchtgehalt dieser Mischung bei 10 bis 40 Gew.-%. Die so erhaltene extrudierbare Masse wird bis zur Homogenität im Mischer gemischt und anschließend einem Extruder zugeführt. Im Extruder wird die Masse durch eine Lochscheibe mit Lochdurchmessern von 0,4 bis 3 mm, vorzugsweise 0,6 mm, zu Strängen extrudiert, die anschließend auf einem Rundungsgerät, z. B. einem Drehtellergerät, zu kugelförmigen Partikeln gerundet werden. Nach der Rundung werden die noch feuchten Partikel in einer Trockenanlage, beispielsweise einer Wirbelschichttrockenanlage, bei einer Temperatur von 30 bis 50 °C bis zu einem Restfeuchtegehalt von 10 bis 2 % getrocknet. Gewünschtenfalls können die erhaltenen Enzymgranulate während dieses Verfahrensschrittes mit einem Schutzlack überzogen werden, um so beispielsweise eine eventuelle Eigenfarbe zu überdecken oder zu verändern. Um hellfarbige Enzymgranulate zu erhalten, können die Enzymgranulate beispielsweise auf an sich bekannte Weise mit einer Titandioxid enthaltenden Dispersion überzogen werden. Hierzu kann auf an sich bekannte Weise Titandioxid mit Polyethylenglykol als Bindemittel in Wasser dispergiert und über Düsen in die Trockenanlage eingespritzt werden.

Nach dem erfindungsgemäßen Verfahren werden Enzymgranulate erhalten, die weitgehend aus abgerundeten staubfreien Partikein mit einem Durchmesser von 0,2 bis 1,0 mm und einem Schüttgewicht von 600 g/l bis 1.100 g/l bestehen und sich vorzugsweise als Bestandteile von pulverförmigen Wasch- und Reinigungsmitteln eignen.

Ein weiterer Gegenstand der Erfindung ist die Verwendung der erfindungsgemäßen Enzymgranulate in pulverförmigen Wasch- und Reinigungsmitteln. Solche Wasch- und Reinigungsmittel können beispielsweise zur Reinigung von Oberflächen, z. B. zur Entfernung von Fettrückständen im Hygiene- oder Lebensmittelbereich, verwendet werden. Bevorzugt werden die erfindungsgemäßen Enzymgranulate in Waschpulverformulierungen zur Reinigung von Textilien oder Geschirr eingesetzt. Außer den Enzymgranulaten können die Waschpulverformulierungen dabei alle an sich im Stand der Technik üblichen Waschmittelinhaltsstoffe wie Tenside, Bleichmittel oder Gerüststoffe (Builder), sowie weitere übliche Hilfsstoffe für die Formulierung von pulverförmigen Waschmitteln in an sich üblichen Mengen enthalten. Zu den Hilfsstoffen gehören z. B. Verstärker, Enzymstabilisatoren, Schmutzträger und/oder Kompatibilisierungsmittel, Komplex- und Chelatbuildner, Schaumregulatoren und Zusatzstoffe wie optische Aufheller, Opazifizierungsmittel, Korrosionsinhibitoren, Antielektrostatika, Farbstoffe, Bakterizide, Bleichmittel, Aktivatoren, Persäurebleichmittelvorstufen. So können neben den erfindungsgemäßen Enzymgranulaten Bleichmittel oder Bleichmittelgemische auf der Basis von Sauerstoffverbindungen, z. B. Perborate wie Natriumperborat-Tetrahydrat oder Natriumperborat-Monohydrat in an sich üblichen Mengen in den Waschpulverformulierungen enthalten sein.

Durch die Einarbeitung von einem Alkali- oder Erdalkali formiat, gegebenenfalls in Kombination mit einem reduzierden Zucker, werden die Enzyme in den erfindungsgemäßen Granulaten in überraschender Weise stabilisiert. So zeigen die erfindungsgemäßen Enzymgranulate sehr gute, für die Verwendung in pulverförmigen Waschmittelformulierungen besonders geeignete Eigenschaften. In pulverförmigen Waschmittelformulierungen weisen sie eine sehr hohe Lagerstabilität auf, wodurch sie insbesondere auch für oxidationsmittelhaltigen Waschmittelformulierungen wie z. B. peroxidhaltigen Waschmittelformulierungen besonders geeignet werden. Auch zeichnen sich die erfindungsgemäßen Enzymgranulate aufgrund ihres sehr niedrigen Staub- und Enzymstaubgehaltes durch eine sehr gute Verarbeitbarkeit aus. Ein weiterer Vorteil liegt darin, daß größere Enzymverluste bei der erfindungsgemäßen Herstellung der Enzymgranulate vermieden werden können. Zudem besitzen die erfindungsgemäßen Enzymgranulate ausgezeichnete Lösungseigenschaften während des Waschvorganges. So werden bereits innerhalb von 2 Minuten mehr als 90 % Enzym aus den erfindungsgemäßen Enzymgranulaten in die Waschlösung freigesetzt, so daß während des Waschvorganges eine sehr lange Einwirkzeit des Enzyms auf den entsprechenden Reinigungsgegenstand, beispielsweise Geschirr oder Textilien, gewährleistet ist.

Die nachfolgenden Beispiele sollen die Erfindung weiter erläutern, ohne sie jedoch in ihrem Umfang zu begrenzen.

### Beispiele

### Beispiel 1: Herstellung eines Enzymgranulats

Im folgenden wird die Herstellung von Enzymgranulaten, welche als Enzymbestandteil eine hochalkalische Protease enthalten, beschrieben.

Die Aktivität der in den Enzymgranulaten verarbeiteten Proteasen wurde in Delft Units (DU) bestimmt. 1000 DU entsprechen der proteolytischen Aktivität, die bei einem Volumen von 1 ml einer 2 %-igen Enzymlösung (w/w) nach Abbau von Casein eine Extinktionsdifferenz (1 cm Lichtweg; 275 nm; Bestimmung gegen Blindprobentest) von 0,400 ergibt.

In der Herstellung der Enzymgranulate wurde ein auf an sich bekannte Weise durch Fermentation von Bacillus alcalophilus gewonnenes wäßriges Enzymkonzentrat einer hochalkalischen Protease mit einem Feststoffgehalt von ca. 37 Gew.-% und einer proteolytischen Aktivität von ca. 950 000 DU/g eingesetzt.

In einem Konusmischer wurde eine Vormischung der pulverförmigen Rezeptbestandteile hergestellt. Bezogen auf den Gesamtfeststoffgehalt der Enzymgranulate wurden hierfür die folgenden Bestandteile miteinander vermischt:

| | |
|---|---|
| wasserunlösliche Füllstoffe: | 23 Gew.-% Cellulose |
| | 11 Gew.-% Kaolin |
| | 6 Gew.-% Calciumcarbonat |
| | 21 Gew.-% Weizenmehl/Stärke-Gemisch (8 Gew.-% Weizenmehl, 13 Gew.-% Stärke) |
| | |
| wasserlösliche Füllstoffe: | 9 Gew.-% Natriumsulfat |
| | |
| Bindemittel: | 11 Gew.-% Polyethylenglykol 3000 |
| | |
| | 0,8 Gew.-% Polyvinylpyrrolidon K-90 |
| | |
| reduzierende Zucker: | 4,5 Gew.-% Lactose |
| Erdalkaliformiat: | 2,2 Gew.-% Calciumformiat |

Das Enzymkonzentrat sowie soviel Wasser wurde der pulverförmigen Vormischung im Konusmischer beigefügt, daß eine homogene extrudierbare Masse entstand. Der Feuchtgehalt dieser so entstandenen Feuchtmischung lag bei ca. 30 Gew.-%.

Die erhaltene homogene Feuchtmischung wurde in einem Extruder überführt. Durch eine Lochmatritze mit Lochdurchmessern von ca. 0,6 mm wurde die Masse zu Strängen verpreßt. Die dabei erhaltenen Strangbruchstücke wurden in ein Drehtellergerät überführt, in dem sie während einer Bearbeitungszeit von ca. 20 Sekunden zu abgerundeten Partikeln geformt wurden. Die gerundeten Partikel wurden anschließend in einem Wirbelschichttrockner auf einen Wassergehalt von ca. 5 % bei einer Temperatur von ca. 48 °C getrocknet. Außerdem wurden sie im Wirbelschichttrockner auf an sich bekannte Weise mit einem Schutzlack, bestehend aus einer wäßrigen Dispersion aus Titandioxid mit Polyethylenglykol als Bindemittel überzogen.

Das erhaltene Granulat bestand aus gerundeten Partikeln mit Durchmessern von 0,2 bis 1,0 nm. Das Granulat war nichtklebend und gut schüttfähig mit einem Schüttgewicht von ca. 800 g/l.

### Beispiel 2 bis 5: Herstellung weiterer Enzymgranulate

Auch in den Enzymgranulaten der Beispiele 2 bis 5 wurden auf an sich bekannte Weise durch Fermentation von Bacillus alcalophilus erhaltene wäßrige Enzymkonzentrate einer hochalkalischen Protease in gleicher Weise verarbeitet. Der Feststoffgehalt dieser Enzymkonzentrate lag bei ca. 11,5 Gew.-%, entsprechend einer proteolytischen Aktivität von ca. 320.000 DU/g. In der nachfolgenden Tabelle sind die Konzentrationen der weiteren Bestandteile, jeweils bezogen auf den Gesamtfeststoffgehalt angegeben.

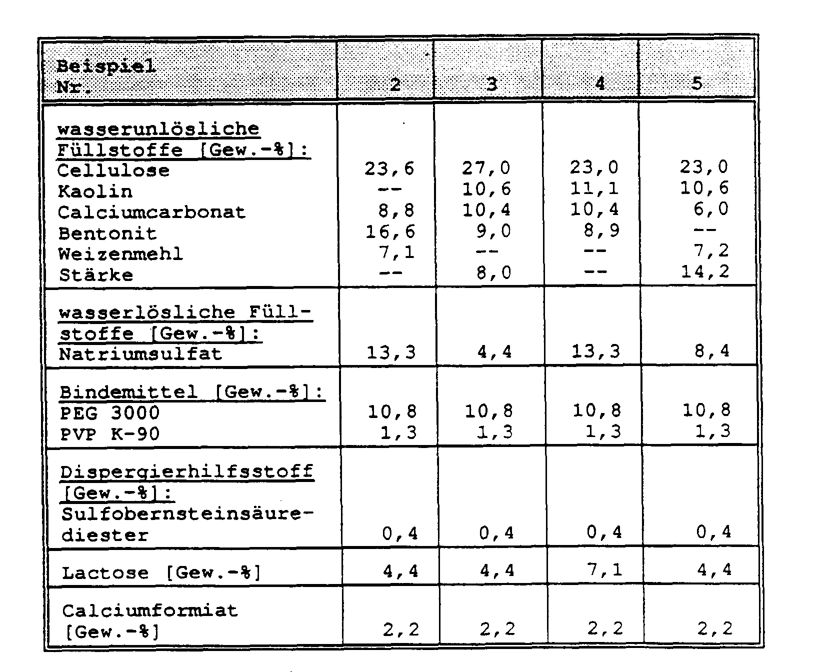

Die nach den Beispielen 2 bis 5 erhaltenen Enzymgranulate wurden ebenfalls, wie bereits für Beispiel 1 beschrieben, mit einem Schutzlack aus Titandioxid mit Polyethylenglykol als Bindemittel überzogen.

### Beispiel 6: Lösungseigenschaften der Enzymgranulate

Die Lösungseigenschaften der erhaltenen Enzymgranulate wurden wie nachfolgend bestimmt.

In einem 400 ml-Becherglas wurden 200 ml einer wäßrigen 2 %-igen Natriumtripolyphosphat-Lösung bei 22 °C mit einem mechanischen Flügelrührer mit einer konstanten Drehgeschwindigkeit von 700 Umdrehungen pro Minute gerührt. Die Lösung hatte einen Wasserhärtegrad von 15 ° Deutsche Härte.

Unter Vermeidung von Klumpenbildung wurden 1 g Enzymgranulat in die gerührte Lösung gegeben. Nach 2, 3 und 5 Minuten wurden Proben entnommen, die über eine Nutsche (Filterpapier: Schleicher und Schüll 589) abgesaugt wurden. In den Filtraten wurde dann die proteolytische Aktivität ermittelt. Die in den Filtraten bestimmte Proteaseaktivität (gemessen in DU) wurde auf die in den zugegebenen Enzymgranulaten enthaltene Enzymaktivität bezogen, wobei die Ausgangsaktivität in 1 g Enzymgranulat 100 % Proteaseaktivität entspricht.

Nach 2 Minuten waren aus dem nach Beispiel 1 hergestellten Ezymgranulaten ca. 90 % Protease aus dem Granulat freigesetzt. Nach 3 Minuten war die Protease zu ca. 96 %, nach 5 Minuten zu 99 % freigesetzt.

### Beispiel 7: Lagerbeständigkeit der Enzymgranulate in Gegenwart von Waschmittelinhaltsstoffen

Zur einer für Waschmittelhersteller kommerziell erhältlichen perborathaltigen Waschmittelbasisformulierung, die 18,4 Gew.-% Zeolith, 7,3 Gew.-% Natriumcarbonat, 4,8 Gew.-% lineares Alkylbenzolsulfonat, 3,3 Gew.-% Nonionics, 3,3 Gew.-% Seife, 0,1 Gew.-% Entschäumer, 1,5 Gew.-% Carboxymethylcellulose, 0,15 Gew.-% optischen Aufheller, 3,8 Gew.-% Natrium-di-silikat, 25 Gew.-% Perborat-tetrahydrat, 1,5 Gew.-% TAED und 30,85 Gew.-% Natriumsulfat enthielt, wurde in einer Menge von 1,0 Gew.-%, bezogen auf die Waschmittelbasisformulierung, erfindungsgemäßes Enzymgranulat beigemischt. Anschließend wurde dieses Gemisch in mit Polyethylen beschichteten Pappkartons (Größe: 7 · 11,8 · 2 cm) abgefüllt, die dann anschließend in einem Klimaschrank bei 35 °C und 80 % relativer Luftfeuchtigkeit gelagert wurden. Nach Beendigung der Lagerzeit wurden den Kartons Proben entnommen, diese in Natriumsulfit-Lösung (10 g/l, pH 8,5) gelöst und mit dieser Lösung auf an sich bekannte Weise die enzymatische Aktivität bestimmt.

Nach einer Lagerzeit von 24 Tagen betrug die enzymatische Aktivität der gelösten enzymatischen Waschmittelformulierung immer noch 76 %, bezogen auf die ursprünglich vorhandene Enzymaktivität in der Waschmittelformulierung, wie sie in einer Messung unter identischen Bedingungen vor den Lagerversuchen für die enzymhaltigen Waschmittelformulierung ermittelt wurde. Dies belegt die hervorragende Lagerbeständigkeit der erfindungsgemäßen Enzymgranulate in Gegenwart von Waschmittelinhaltsstoffen.

## Patentansprüche

1. Enzymgranulat, enthaltend ein Enzym oder Enzymgemisch, ein wasserunlösliches Füllstoffgemisch, wasserlösliche Füllstoffe, Bindemittel sowie gegebenenfalls weitere Granulierhilfsstoffe, dadurch gekennzeichnet, daß es ein Alkali- oder Erdalkaliformiat und gegebenenfalls reduzierende Zucker enthält.

2. Enzymgranulat gemäß Anspruch 1, dadurch gekennzeichnet, daß es Calciumformiat enthält.

3. Enzymgranulat gemäß Anspruch 1, dadurch gekennzeichnet, daß es als reduzierende Zucker reduzierende Disaccaride, vorzugsweise Lactose und/oder Maltose, enthält.

4. Enzymgranulat gemäß Anspruch 1, dadurch gekennzeichnet, daß es als Enzym eine Protease, Amylase, Cellulase, Hemicellulase, Lipase, Oxidoreduktase oder deren Gemische enthält.

5. Enzymgranulat gemäß Anspruch 1, dadurch gekennzeichnet, daß es als wasserunlösliches Füllstoffgemisch ein Gemisch aus Cellulose, einem Schichtsilikat oder Schichtsilikatgemisch und gegebenenfalls Getreidemehl und/oder Stärke enthält.

6. Enzymgranulat gemäß Anspruch 1, dadurch gekennzeichnet, daß es als wasserlösliche Füllstoffe Alkalichloride oder Alkaliacetate oder Alkalisulfate oder deren Gemische enthält.

7. Enzymgranulat gemäß Anspruch 1, dadurch gekennzeichnet, daß es als Bindemittel Polyethylenglykol mit Molekulargewichten im Bereich 200 bis 10.000 und Polyvinylpyrrolidon mit Molekulargewichten im Bereich 12.000 bis 3.000.000 enthält.

8. Enzymgranulat gemäß Anspruch 1, dadurch gekennzeichnet, daß es aus Partikeln mit einer Korngröße im Bereich von 0,2 bis 1,0 mm besteht.

9. Verwendung von Enzymgranulat gemäß einem der vorherigen Ansprüche in pulverförmigen Wasch- und Reinigungsmitteln.

10. Verfahren zur Herstellung eines Enzymgranulates, wobei man eine durch Vermischen eines Enzymkonzentrates mit Wasser, wasserlöslichen und wasserunlöslichen Füllstoffen, Bindemitteln sowie gegebenenfalls weiteren Granulierhilfsstoffen erhaltene extrudierbare Masse zu Partikeln extrudiert, die erhaltenen Partikel in einem Rundungsgerät rundet, die gerundeten Partikel anschließend trocknet sowie gegebenenfalls mit einem Schutzlack überzieht, dadurch gekennzeichnet, daß man der extrudierbaren Masse, bezogen auf den Gesamtfeststoffgehalt des Enzymgranulates, ein Alkali- oder Erdalkaliformiat in einer Menge von 0,5 bis 15 Gew.-% und gegebenenfalls reduzierende Zucker in einer Menge von 0,5 bis 20-Gew.-% zugibt.

## Claims

1. Enzyme granules comprising an enzyme or enzyme mixture, a water-insoluble filler mixture, water-soluble fillers, binders and, if appropriate, other granulating auxiliaries, characterized in that they comprise a formate of an alkali metal or alkaline earth metal and if appropriate reducing sugars.

2. Enzyme granules according to Claim 1, characterized in that they comprise calcium formate.

3. Enzyme granules according to Claim 1, characterized in that they comprise reducing disaccharides, preferably lactose and/or maltose, as the reducing sugars.

4. Enzyme granules according to Claim 1, characterized in that they comprise a protease, amylase, cellulase, hemicellulase, lipase or oxidoreductase or mixtures thereof as the enzyme.

5. Enzyme granules according to Claim 1, characterized in that they comprise a mixture of cellulose, a laminar silicate or laminar silicate mixture and if appropriate cereal flour and/or starch as the water-insoluble filler mixture.

6. Enzyme granules according to Claim 1, characterized in that they comprise alkali metal chlorides or alkali metal acetates or alkali metal sulphates or mixtures thereof as the water-soluble fillers.

7. Enzyme granules according to Claim 1, characterized in that they comprise polyethylene glycol having molecular weights in the range of 200 to 10,000 and polyvinylpyrrolidone having molecular weights in the range of 12,000 to 3,000,000 as the binders.

8. Enzyme granules according to Claim 1, characterized in that they consist of particles having a particle size in the range from 0.2 to 1.0 mm.

9. Use of enzyme granules according to one of the preceding claims in pulverulent detergents and cleaning agents.

10. Process for the preparation of enzyme granules, in which an extrudable composition obtained by mixing an enzyme concentrate with water, water-soluble and water-insoluble fillers, binders and, if appropriate, other granulating auxiliaries is extruded to particles, the resulting particles are rounded in a rounding apparatus and the rounded particles are then dried and, if appropriate, coated with a protective coating, characterized in that a formate of an alkali metal or alkaline earth metal in an amount of 0.5 to 15% by weight and, if appropriate, reducing sugars in an amount of 0.5 to 20% by weight, based on the total solids content of the enzyme granules, are added to the extrudable composition.

## Revendications

1. Granulat d'enzymes, comprenant une enzyme ou un mélange d'enzymes, un mélange insoluble de matières de remplissage, des matières de remplissage solubles, des agents liants, ainsi qu'optionnellement d'autres matières aidant à la granulation, caractérisé en ce qu'il contient un formiate alcalin ou alcalino-terreux et optionnellement des sucres réducteurs.

2. Granulat d'enzymes selon la revendication 1, caractérisé en ce qu'il contient un formiate de calcium.

3. Granulat d'enzymes selon la revendication 1, caractérisé en ce qu'il contient, comme sucres réducteurs, des disaccharides réducteurs, et préférentiellement du lactose et/ou du maltose.

4. Granulat d'enzymes selon la revendication 1, caractérisé en ce qu'il contient, comme enzyme, une protéase, une amylase, une cellulase, une hémicellulase, une lipase, une oxydoréductase, ou leurs mélanges.

5. Granulat d'enzymes selon la revendication 1, caractérisé en ce qu'il contient, comme mélange insoluble de matières de remplissage, un mélange de celluloses, un phyllosilicate ou un mélange de phyllosilicates, et optionnellement de la farine de céréales et/ou de l'amidon.

6. Granulat d'enzymes selon la revendication 1, caractérisé en ce qu'il contient, comme matières de remplissage solubles, des chlorures alcalins, des acétates alcalins ou des sulfates alcalins, ou leurs mélanges.

7. Granulat d'enzymes selon la revendication 1, caractérisé en ce qu'il contient, comme agents liants, des polyethylèneglycols dont la masse moléculaire est comprise dans le domaine 200 à 10 000 et des polyvinylpyrrolidones dont la masse moléculaire est comprise dans le domaine 12 000 à 3 000 000.

8. Granulat d'enzymes selon la revendication 1, caractérisé en ce qu'il est constitué de particules dont la granulométrie est comprise dans le domaine de 0,2 à 1,0 mm.

9. Utilisation d'un granulat d'enzymes selon l'une des revendications précédentes dans les produits de lavage ou de nettoyage sous forme de poudre.

10. Procédé d'obtention d'un granulat d'enzymes, par extrusion de particules d'une pâte extrudable obtenue par le mélange d'un concentré d'enzymes avec de l'eau, des matières de remplissage solubles et insolubles, des agents liants, ainsi qu'optionnellement d'autres matières aidant à la granulation, arrondissage des particules ainsi obtenues dans un appareil à arrondir, puis séchage et optionnellement enduisage par un vernis protecteur des particules arrondies, caractérisé en ce que l'on ajoute à la pâte extrudable, en proportion relative au contenu total en matière solide du granulat d'enzymes, 0,5 à 15 % en poids d'un formiate alcalin ou alcalino-terreux et optionnellement 0,5 à 20 % en poids de sucres réducteurs.
